# EUROPEAN PATENT SPECIFICATION

(11) **EP 3 004 854 B1**
(45) Date of publication and mention of the grant of the patent: **08.07.2020**
(21) Application number: 14730951.2
(22) Date of filing: 22.05.2014
(51) Int. Cl.: G01N 27/04, A61F 13/02, A61F 13/00, A61F 13/42

(54) **MOISTURE INDICATING SYSTEM**
FEUCHTIGKEITSANZEIGESYSTEM
SYSTÈME INDICATEUR D'HUMIDITÉ

(30) Priority: 24.05.2013 GB 201309369
(43) Date of publication of application: 13.04.2016
(73) Proprietor: Smith & Nephew plc, Watford, Hertfordshire WD18 8YE (GB)
(72) Inventor: HARTWELL, Edward Yerbury, York Yorkshire YO10 5DF (GB); HICKS, John Kenneth, York Yorkshire YO10 5DF (GB)
(74) Representative: Smith & Nephew
(86) International application number: PCT/GB2014/051574
(87) International publication number: WO 2014/188200

(56) References cited:
- WO-A1-02/47737
- WO-A1-2012/074509
- US-A1- 2013 131 621

## Description

### Background

The field of wound care management has long understood that keeping wounds optimally moist can help the cells in the wound area grow and migrate to the proper location to help the wound heal. Achieving an optimal moist environment relies on good clinical judgement to determine the correct moisture levels, since too little moisture can desiccate the wound and too much can lead to maceration of the wound bed and surrounding tissue. It is therefore important to be able to monitor moisture, to properly and optimally change the bandage, while still allowing the wound to heal undisturbed. Common techniques for performing such monitoring rely on visual indications of excess moisture or strikethrough on wound dressings. Commonly used indications include visual changes on backing materials or leakage from the dressing.

Some current wound dressings utilize an indicator layer containing a dye which changes colour on contact with wound exudate. A typical dye is gentian violet, which changes from violet to purple when wet, indicating that the dressing is saturated. That change is typically hard to perceive and therefore users often find it unreliable. Other current wound dressings, for example the DuoDerm® Signal dressing sold by Convatec, rely on fluid leaking from the wound into an area behind an impermeable outer covering of the dressing, causing a blister to become visible. Once the edge of the blister reaches an indicator line marked on the outer surface of the dressing changing is required. As the indicator is merely a blister on the surface ofthe dressing, it is often difficult to read. Additionally, the blister can enlarge and fill with fluid that exceeds the requirements of a healing environment and fosters an environment for bacterial colonization. There is a need in the art for a moisture indicating wound dressing in which the moisture indicator within the dressing provides more sensitive moisture detection with a more ascertainable signal to the user.
US 2013/131621 discloses a transparent, absorbent device for the dressing of wounds and insertion sites of percutaneous and drug delivery devices.
WO 2012/074509 discloses methods, devices and systems for patch based physical, physiological, chemical, and biochemical sensors that diagnose and monitor disease states.
WO 02/47737 discloses a layered wound dressing material comprising: a wound facing hydrogel layer and a barrier layer, wherein the barrier layer comprises a pH-sensitive material that is substantially insoluble in water at 25°C under acidic conditions, but substantially soluble in water at 25°C under neutral or alkaline conditions.

### Summary

This application discloses devices and methods related to wound dressings having moisture indicators. The underlying mechanism for monitoring the wound exudate loading within a wound dressing utilises a colour change of a pH indicator provided within the dressing. The colour change is driven by the increased acidity or alkalinity of a wound exudate as it migrates through the dressing. This alteration in the pH of the wound exudate is a consequence of the exudate dissolving a soluble composition which is provided within the dressing and which consequently releases hydrogen or hydroxide ions into the wound exudate. The modified wound exudate, loaded with the hydrogen or hydroxide ions, has a pH which is either more acidic or more alkaline than the unmodified wound exudate. As a result, the modified wound exudate causes a more amplified change in the colour of the pH indicator than would be caused by the unmodified wound exudate. A device is disclosed which includes (i) a soluble composition capable of releasing hydrogen or hydroxide ions upon solubilisation and (ii) a colour-based pH indicator, wherein the colour ofthe pH indicator is correlated to a pH. The device can be used in combination with a conventional wound dressing. Alternatively, the device can be manufactured as a component of a wound dressing. The colour change of the pH indicator provides a visual indication of the wound exudate loading within the dressing. A colour change is indicative of the wound exudate reaching the part ofthe dressing where the device is located.
The invention is defined by the wound dressing of independent claim 1. Optional features of the invention are provided in the dependent claims.

In one aspect, a device includes a first composition which has a first colour and which changes to a second colour in response to a change in pH and a second composition which dissolves upon contact with a wound exudate to release hydrogen or hydroxide ions, the wound exudate loaded with the released hydrogen or hydroxide ions interacts with the first composition to cause the change to the second colour. In certain aspects, the first and second compositions are impregnated into different carrier materials within the device. In certain aspects, the carrier materials are physically separated by a spacer material. In certain aspects, the first and second compositions are impregnated into the same carrier material within the device. In certain aspects, the first and second compositions which are impregnated within the same carrier material are physically separated, for example, at least one of the first or second compositions is encapsulated in a soluble barrier which dissolves upon contact with wound exudate to enable interaction between the compositions.

In another aspect, a wound dressing is disclosed which includes a device which indicates wound exudate loading. The device includes: a first composition which has a first colour and which changes to a second colour in response to pH, and a second composition which dissolves upon contact with a wound exudate to release hydrogen or hydroxide ions into the wound exudate and wherein the wound exudate loaded with the hydrogen or hydroxide ions causes the first composition to change to the second colour. In certain aspects, the wound dressing includes an absorbent layer which has a wound-facing surface and an opposing non-wound-facing surface, and the device is positioned within the dressing such that the device is in contact with the non-wound-facing surface of the absorbent layer. In certain aspects, the wound dressing has a peripheral edge and the device extends outwardly from the peripheral edge. In certain aspects, the device forms an annular flange or annular ring, which partially or fully encircles the peripheral edge ofthe dressing.

In another aspect, a device is disclosed which indicates wound exudate loading within a wound dressing. The device includes a pH-dependent moisture indicating means and a means of generating ions. Contact between the wound exudate and the means of generating results in a wound exudate which is loaded with ions and which interacts with the pH-dependent moisture indicating means to cause a visual change. In certain aspects, the ions generated are hydrogen or hydroxide ions. In certain aspects, the visual change is a colour change from a first colour to a second colour.

In a further aspect, a device is disclosed which indicates wound exudate loading within a wound dressing, the device includes a first composition which transforms from a first state to a second state and a second composition which dissolves upon contact with the wound exudate and which forces the transformation of the first composition from the first state to the second state upon contact therewith. In certain embodiments, the first state is a first colour and the second state is a second colour. In certain embodiments, the second composition releases ions into the wound exudate to alter analyte levels within the wound exudate. In certain embodiments, the wound exudate with released ions causes a change to the second state.

In another aspect, methods are disclosed for monitoring loading of a wound dressing by a wound exudate. The methods include steps of (a) locating a first composition within the wound dressing, wherein the first composition has a first colour prior to contact with the wound exudate and which changes to a second colour in response to a pH change; (b) locating a second composition within the wound dressing, wherein the second composition dissolves upon contact with a wound exudate to release hydrogen or hydroxide ions into the wound exudate; (c) applying the wound dressing to the wound; (d) contacting the second composition with the wound exudate as the wound exudate passes through the dressing, thereby dissolving the second composition and releasing hydrogen or hydroxide ions into the wound exudate; and (e) contacting the first composition with the wound exudate loaded with the released hydrogen or hydroxide ions to cause the first composition to change to the second colour and wherein the development of the second colour indicates that the wound dressing is saturated at the location of the second composition prior to its dissolution. The methods further include the step of removing the dressing when the second colour becomes visible.

In another aspect, methods are disclosed for monitoring loading of a wound dressing by a wound exudate. The methods utilise the dissolution of a soluble component provided at a location with the wound dressing by the wound exudate to cause a component which has a first state prior to contact with the wound exudate loaded with the solubilised component to change to a second state upon said contact, thereby indicating that the wound dressing in saturated at the location of the soluble component.

In further aspects, methods are disclosed for monitoring loading of a wound dressing by a wound exudate, the methods include steps of: (a) providing a wound dressing containing a first composition that can be solubilised into ions and a second composition containing a pH indicator that indicates a pH change; (b) flowing wound exudate into contact with the dressing to solubilise the ions; and (c) contacting the second composition with the solubilised ions until a pH is indicated.

Further areas of applicability of the disclosed devices and methods will become apparent from the detailed description provided hereinafter. It should be understood that the detailed description and specific examples, while indicating particular embodiments, are intended for purposes of illustration only and are not intended to limit the scope of the disclosure or any of the claims that may be pursued.

### Description of the drawings

The foregoing and other objects and advantages will be appreciated more fully from the following further description thereof, with reference to the accompanying drawings. These depicted embodiments are to be understood as illustrative and not limiting in any way:
Figures 1A and 1B are side cross-sectional views of an illustrative device in which the colour of a pH indicator changes from a first colour to a second colour.
Figures 2A and 2B are side cross-sectional views of the device illustrated in Figures 1A and 1B having a spacer layer to physically separate a soluble composition and a pH indicator.
Figures 3A and 3B are side cross-sectional views of an illustrative device, in which a soluble composition is encapsulated with a soluble barrier.
Figures 4A and 4B are side cross-sectional views of an illustrative combination of a wound dressing and a device as disclosed herein.
Figures 5A and 5B are plan views of an illustrative combination of a wound dressing and a device as disclosed herein.

### Detailed Description

To provide an understanding of the devices and methods describe herein, certain illustrative embodiments will now be described. For the purpose of clarity and illustration, the devices herein are described as having a pH-dependent moisture indicator which changes colour in response to a pH change.

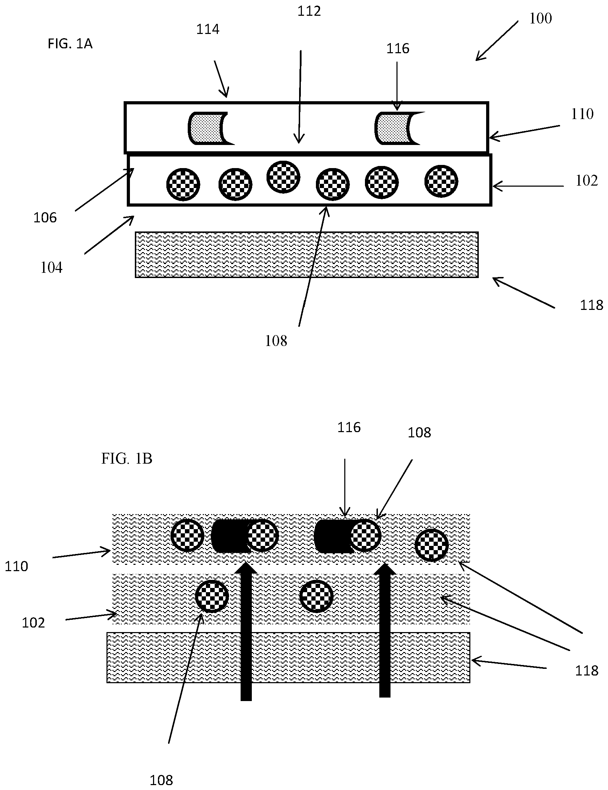

Figure 1A depicts a device 100 which is designed to be incorporated into a wound dressing in order to indicate the wound exudate loading of the dressing, as will be explained in greater detail below. Device 100 has a first carrier material 102, which can be any material that is permeable to liquid, such as wound exudate. For example, the first carrier material 102 can be a cellulosic material. A suitable cellulosic material is conventional filter paper. The first carrier material 102 has a wound facing surface 104 and an opposing non-wound facing surface 106. The first carrier material 102 is impregnated with a soluble composition 108 which is a source of hydrogen or hydroxide ions, upon solubilisation by wound exudate. In certain embodiments, the source of hydrogen ions is an acid, such as citric acid. In alternative embodiments, the source of hydroxide ions is an alkali, such as sodium carbonate.

The device also includes a second carrier material 110 which has a wound facing surface 112 and an opposing non-wound facing surface 114. The wound facing surface 112 is located on or adjacent to the non-wound facing surface 106 of the first carrier material 102. In certain implementations, the two surfaces 112 and 106 form a composite. The second carrier material 110 is preferably white, although it is envisaged that other colours could be utilised. The second carrier material 110 is impregnated with a pH indicator 116 which has a first colour and which can change to a second colour upon interaction with hydrogen or hydroxide ions. The second colour is indicative of the pH of the ion-loaded wound exudate. Suitable pH indicators that indicate pH based upon colour are readily apparent to persons skilled in the art, and include for example, a universal indicator solution.

As shown in Figure 1B, when wound exudate 118 migrates into the first carrier material 112, the soluble composition 108 is at least partially solubilised to release hydrogen or hydroxide ions into the wound exudate. The ions are carried within the wound exudate into the second carrier material 110 as the wound exudate migrates through the dressing. Within the first carrier material 103 the ions interact with the pH indicator. This interaction causes the pH indicator to change from the first colour to the second colour. Because the ion-loaded wound exudate has a pH which is more acidic or more alkaline than the endogenous, unmodified, wound exudate there is a greater shift in the colour of the pH indicator than would occur with endogenous wound exudate. This colour change is thus amplified and easier to perceive by the user. A consideration to be made when choosing the species and concentration of the soluble solution to use in the device is that the release of hydrogen or hydroxide ions into the wound exudate should preferably result in the pH of the wound exudate becoming either very acidic (for example, pH 0, pH1, pH 2, pH3) or very alkaline (for example, pH 11, pH12, pH13 or pH14) as changes to these pH's produce colours which are less ambiguous to the patient and/or clinician than colours in the intervening pH range. Also preferred is to minimise the neutralisation of the hydrogen and hydroxide ions by the endogenous wound exudate. It is therefore envisaged that a range of devices can be available, each having different combinations of soluble compositions (e.g., species and concentrations) and pH indicators. This enables the clinician to tailor the use of the device to the clinical situation, for example the wound type. The clinician can test the pH of the endogenous wound fluid and choose the appropriate device accordingly, that is the device which will provide the most perceivable shift in colour upon contact with the ion-loaded wound exudate.

In certain embodiments, the source of hydrogen ions is citric acid and the pH indicator is a universal indicator solution. The universal indicator is loaded onto a cellulosic second carrier material 110 at neutral pH and has a first colour of yellow/orange. The hydrogen ions released by the solubilisation of the citric acid interact with the universal indicator, forcing a change to the second colour. The second colour is red.

In certain embodiments, the source of hydroxide ions is sodium carbonate and the pH indicator is universal indicator. The universal indicator is loaded onto a cellulosic second carrier material 110 at neutral pH and has a first colour of yellow/orange. The hydroxide ions released by the sodium carbonate interact with the universal indicator, forcing a change to the second colour. The second colour is violet/purple.

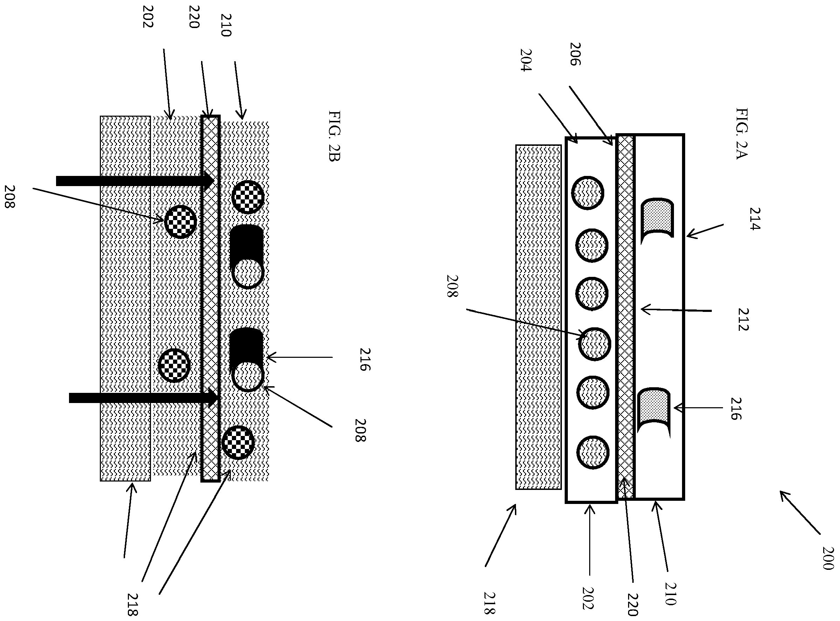

Figures 2A and 2B depict device 200 which is similar to the device illustrated in Figures 1A and 1B and which additionally includes a spacer layer 220 positioned between the first carrier material 202 and the second carrier material 210. The device is designed to be incorporated into a wound dressing in order to indicate the wound exudate loading of the dressing. Wound dressings are sterilised before use, and one conventional means of sterilisation uses ethylene oxide. A typical treatment protocol contains a high humidity cycle and this risks some degree of premature solubilisation of the soluble composition 208 due to contact with the moisture. The premature solubilisation and consequent interaction of the ions with the pH indicator would be detrimental to the functionality of the product, leading to false results. For example, it could appear that the dressing is saturated, when in fact it is not, leading to unnecessary dressing changes. Minimising premature interaction between the soluble composition and the pH indicator is therefore desirable. This is achieved by physically separating these components within the device so that even if some degree of solubilisation of the soluble composition results from the sterilisation protocol, the ions released are unable to sufficiently interact with the pH indicator to cause a colour change. The spacer layer 220 is therefore incorporated into the device to physically separate the pH indicator 216 and the source of hydrogen or hydroxide ions 208.

The spacer layer 220 can be made of any material that allows a wound exudate which is loaded with ions to migrate through towards the second carrier material. The type of material and its morphology can be chosen in order to tune the moisture level that is required to trigger the indicator system. In some embodiments the spacer layer 220 is a cellulose-based paper, for example, a conventional filter paper. In certain embodiments, the spacer layer 220 is a 3-D fabric, for example, the spacer layer can be a knitted or woven spacer fabric, such as Baltex 7970 weft knitted polyester. In certain embodiments, the spacer layer is a non-woven fabric.

In certain embodiments, the spacer layer is a composite which utilises the differential between filament counts to promote and direct the transport of wound exudate upwards through the device. For example, the spacer layer can consist of a layer of knitted polyester viscose, cellulose or other monofilament fiber which is sandwiched between an upper layer of 84/144 textured polyester and a lower layer of 100 denier flat polyester.

In order to promote wound exudate transport through the spacer layer, the material of the spacer layer is advantageously hydrophilic. In certain embodiments, the material is inherently hydrophilic. Alternatively, a hydrophilic coating can be applied to the material in order to increase the hydrophilic nature of the material. Alternatively, treatments to increase the hydrophilic nature of the material, for example by removing any manufacturing products such as mineral oils, fats and/or waxes may be utilised. Suitable cleaning treatments may include washing with dry cleaning agents, such as perchloroethylene and/or aqueous cleaning agents such as ionic and non-ionic detergents in aqueous solution. Optionally, an additional manufacturing step can subsequently be carried out in which the 3D spacer fabric is washed in a hydrophilic agent (such as, but not limited to, Feran Ice 30g/l available from the Rudolph Group).

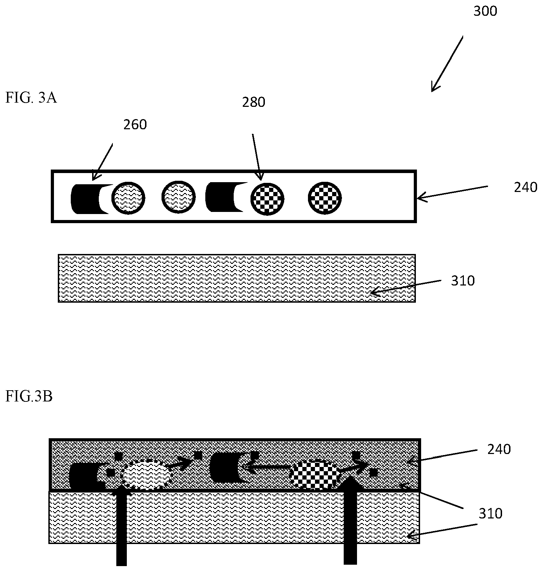

Figures 3A depicts an alternative embodiment, device 300 which has a single layer of carrier material 240 that is impregnated with both a soluble composition 260 and a pH indicator 280. In order to prevent premature interaction of the components prior to use on a wound, for example during ethylene oxide sterilisation protocols, these components can be physically separated. This physical separation is temporary. A suitable mechanism of separation includes encapsulating at least one of the components in a resorbable coating, spacing the components in the fabric, or otherwise spacing them within the layer. In Figure 3A, the soluble composition 260 is shown as being encapsulated in a resorbable coating. In alternative embodiments, the pH indicator 280 is encapsulated in a resorbable coating. In further alternative embodiments, both the soluble composition 260 and the pH indicator 280 are encapsulated in a resorbable coating. Suitable resorbable coatings are readily apparent to persons skilled in the art. As illustrated in Figure 3B, when wound exudate 310 migrates into the device, the resorbable coating dissolves, causing the wound exudate to dissolve the soluble composition 260, thereby releasing the hydrogen or hydroxide ions into the wound exudate. The wound exudate loaded with hydrogen or hydroxide ions interacts with the pH indicator to cause a change in the colour of the pH indicator from a first colour to a second colour. This colour change is indicative that wound exudate has contacted the soluble composition within the device 300.

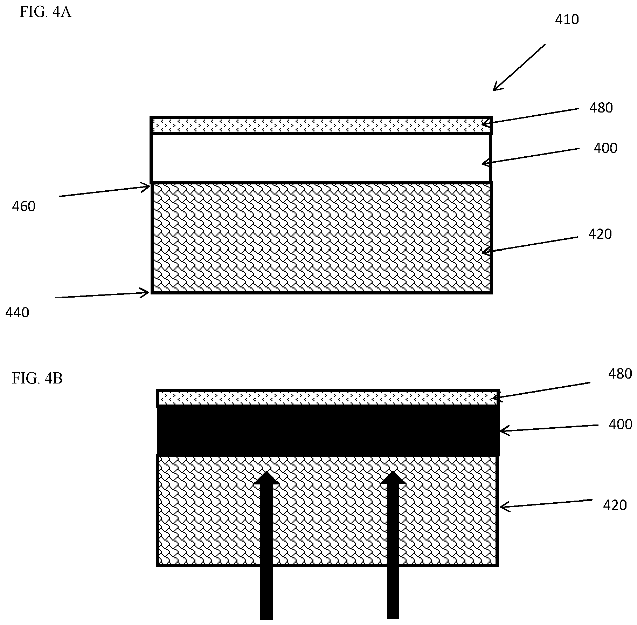

Figure 4A illustrates a device/wound dressing composite 410 having a device 400 for indicating the wound exudate loading of the wound dressing combined with a conventional wound dressing. The device 400 can be one of the embodiments of the device 100, 200 or 300 as described above and as illustrated in Figure 1A, 2A or 3A, or a variant thereof. The wound dressing comprises an absorbent layer 420 which has a wound facing surface 440 and an opposing non-wound facing surface 460. The device 400 is placed near or adjacent to the opposing non-wound facing surface 460 of the absorbent layer 420. The device has a first colour. A barrier layer 480 is used to cover the device to provide additional protection to the wound dressing. Preferably, this barrier layer is made of a material which permits the colour of the pH indicator to be visible therethrough. For example, the barrier layer is made of a substantially transparent material. As illustrated in Figure 4B, when wound exudate (as indicated by the arrows) migrates through the absorbent layer 420 and reaches the device 400 it causes the dissolution of a soluble composition and the consequent release of hydrogen or hydroxide ions into the wound exudate. The interaction of the wound exudate, loaded with the hydrogen or hydroxide ions, with a coloured pH indicator results in a change in the colour of the pH indicator from a first colour to a second colour. This change to a second colour indicates to the patient or clinician that the wound dressing is saturated at the region of the dressing where the device is located, which, in this embodiment, is at the opposing non-wound facing surface 460 of the absorbent layer.

An example method for fabricating a specific embodiment of the structure illustrated in Figures 2A and 2B is outlined below:

In a first step, a first layer is prepared by applying a solution of universal indicator, diluted by 50% with ethanol, to a piece of Whatman No. 1 filter paper (2cm x 2cm) (Sigma-Aldrich) until the paper is saturated. The paper is oven-dried overnight at about 40°C.

In a second step, a second layer is prepared by applying a solution of a 2% solution of aqueous sodium carbonate to a second piece of Whatman No. 1 filter paper (2cm x 2cm) (Sigma-Aldrich) until the paper is saturated. The paper is oven-dried overnight at about 40°C.

In a third step, a three-layered composite is prepared. The upper layer is the first layer, as formed in step 1. The lower layer is the second layer, as formed in step 2. A third, non-treated piece of filter paper is sandwiched between the upper and lower layers to provide a spacer layer. This spacer layer ensures that the sodium carbonate and universal indicator are physically separated, and thereby prevents any soluble sodium carbonate (formed during ethylene oxide sterilisation process) from prematurely interacting with the universal indicator. The three layers are held together by a porous adhesive, the pores permitting migration of wound exudate.

In certain embodiments, the filter paper which functions as a spacer layer is replaced with a 3-D spacer layer, for example, the spacer layer utilised within the PICO product (Smith & Nephew).

An example method for fabricating a specific embodiment of the structure illustrated in Figures 4A and 4B is outlined below:

In a first step, a three-layered composite device as described above in relation to Figures 2A and 2B is prepared. The lower layer, which is impregnated with the sodium carbonate, is adhered to the upper surface of an absorbent layer. An adhesive barrier film is applied to the upper surface of the upper layer, the upper layer being impregnated with universal indicator.

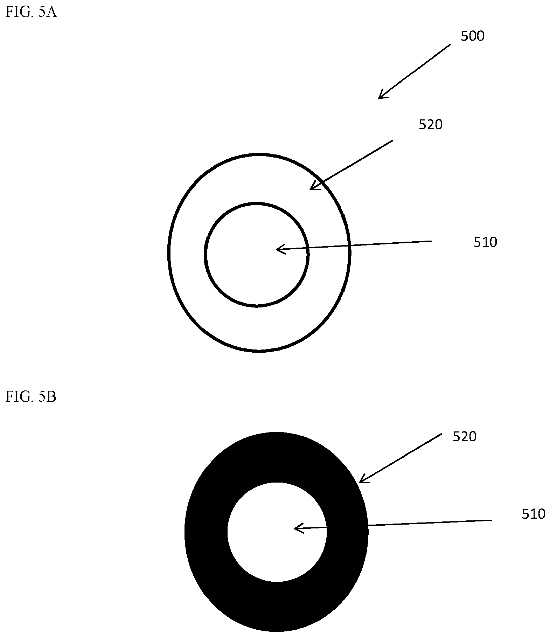

The devices described herein can be manufactured as a separate element to a wound dressing and combined with a conventional wound dressing by the clinician. Alternatively, the devices can be incorporated into a wound dressing at the point of manufacture. In either case, the devices can be located at different regions of the wound dressing to suit the needs of a particular clinical requirement. As illustrated in Figure 5A, the device 520 forms an annular ring which extends from and around a peripheral edge of a wound dressing 510. Upon contact with wound exudate, which migrates to the peripheral edge of the device, the device changes from a first colour to a second colour. Dependent upon the positioning of this device, the patient or clinician is informed, by means of a colour change, that the layer of the dressing from which the device extends is loaded with wound exudate.

There is described herein a device for indicating wound exudate loading within a wound dressing. This device comprises a first composition which transforms from a first state to a second state and a second composition which dissolves upon contact with the wound exudate and which forces the transformation of the first composition from the first state to the second state upon contact therewith. The appearance of the second state is indicative of the level of wound exudate loading and the patient and/or clinician can make an informed decision as to whether to change the dressing. In the embodiments of the device described above, the first composition is a pH indicator which displays a colour that correlates with a pH. The first state is a first colour and the second state is a second colour. In the embodiment of the device described above, the second composition is a soluble composition which releases hydrogen or hydroxide ions when it dissolves upon contact with wound exudate. It is contemplated that within the scope of this application alternative compositions can be utilised which interact by different mechanisms to those described above. The underlying principle is that the second composition is altered by contact with wound exudate and that this altered second composition causes a transformation of the first composition from a first state to a second state. This second state is perceivable to the patient and/or clinician and is indicative of the moisture loading of the wound dressing.

In certain embodiments, the first composition is potassium thiocyanate and the second composition is a soluble iron (III) compound, for example, iron (III) sulphate. The first state is a first colour and the second state is a second colour. The first state of the potassium thiocyanate is colourless. Upon contact with wound exudate, ions released from the soluble iron (III) compound interact with the potassium thiocyanate, forcing a transformation to the second state, that is the second colour. The second colour is red. This second state is perceivable to the patient and/or clinician and is indicative of the moisture loading of the wound dressing.

It is to be understood that the foregoing description is merely illustrative and is not to be limited to the details given.

## Claims

1. A wound dressing comprising an indicator of wound exudate loading within the wound dressing, the indicator comprising a device (100, 200, 300, 400, 520) comprising:
a first composition (116, 216, 280), which transforms from a first state to a second state, each having a different colour; and
a second composition (108, 208, 260), which dissolves upon contact with the wound exudate and which forces the transformation of the first composition from the first state to the second state upon contact therewith,
wherein the second composition (108, 208, 260) releases hydrogen or hydroxide ions into the wound exudate to alter analyte levels within a portion of the wound exudate, and cause the colour change in the first state to the second state.

2. The wound dressing according to claim 1, wherein the first and second compositions are impregnated into different carrier materials within the device.

3. The wound dressing according to claim 2, wherein the first and second carrier materials are physically separated by a spacer material.

4. The wound dressing according to claim 1, wherein the first and second compositions are impregnated into a single carrier material within the device.

5. The wound dressing according to claim 4, wherein at least one of the first or second compositions is encapsulated in a soluble barrier which dissolves upon contact with wound exudate to enable interaction between the compositions.

6. A wound dressing according to claim 1, wherein the wound dressing comprises an absorbent layer having a wound-facing surface and an opposing non-wound-facing surface and wherein the indicator is positioned within the dressing such that the indicator is in contact with the non-wound-facing surface of the absorbent layer.

7. The wound dressing according to any one of claims 1 or 6, wherein the wound dressing has a peripheral edge and wherein the indicator extends outwardly from the peripheral edge.

8. A wound dressing according to claim 7, wherein the indicator extends from the peripheral edge of the dressing to form an annular ring.

## Patentansprüche

1. Ein Wundverband, beinhaltend einen Indikator der Wundexsudatbeladung innerhalb des Wundverbands, wobei der Indikator eine Vorrichtung (100, 200, 300, 400, 520) beinhaltet, die Folgendes beinhaltet:
eine erste Zusammensetzung (116, 216, 280), die sich von einem ersten Zustand in einen zweiten Zustand umgestaltet, wobei jeder eine unterschiedliche Farbe aufweist; und
eine zweite Zusammensetzung (108, 208, 260), die sich bei Kontakt mit dem Wundexsudat auflöst und die die Umgestaltung der ersten Zusammensetzung von dem ersten Zustand in den zweiten Zustand bei Kontakt damit erzwingt,
wobei die zweite Zusammensetzung (108, 208, 260) Wasserstoff- oder Hydroxidionen in das Wundexsudat freisetzt, um Analytspiegel innerhalb eines Teils des Wundexsudats zu verändern und die Farbänderung in dem ersten Zustand zu dem zweiten Zustand zu verursachen.

2. Wundverband gemäß Anspruch 1, wobei die erste und zweite Zusammensetzung in unterschiedliche Trägermaterialien innerhalb der Vorrichtung imprägniert sind.

3. Wundverband gemäß Anspruch 2, wobei das erste und zweite Trägermaterial durch ein Beabstandungsmaterial physikalisch getrennt sind.

4. Wundverband gemäß Anspruch 1, wobei die erste und zweite Zusammensetzung in ein einziges Trägermaterial innerhalb der Vorrichtung imprägniert sind.

5. Wundverband gemäß Anspruch 4, wobei mindestens eine der ersten oder zweiten Zusammensetzung in einer löslichen Barriere eingekapselt ist, die sich bei Kontakt mit Wundexsudat auflöst, um Wechselwirkung zwischen den Zusammensetzungen zu ermöglichen.

6. Wundverband gemäß Anspruch 1, wobei der Wundverband eine absorbierende Schicht mit einer wundzugewandten Oberfläche und einer gegenüberliegenden nicht wundzugewandten Oberfläche beinhaltet und wobei der Indikator innerhalb der Verbands positioniert ist, sodass der Indikator in Kontakt mit der nicht wundzugewandten Oberfläche der absorbierenden Schicht ist.

7. Wundverband gemäß einem der Ansprüche 1 oder 6, wobei der Wundverband einen Umfangsrand aufweist und wobei der Indikator sich von dem Umfangsrand nach außen erstreckt.

8. Wundverband gemäß Anspruch 7, wobei der Indikator sich von dem Umfangsrand des Verbands erstreckt, um einen ringförmigen Ring zu bilden.

## Revendications

1. Un pansement pour plaie comprenant un indicateur de remplissage d'exsudat de plaie à l'intérieur du pansement pour plaie, l'indicateur comprenant un dispositif (100, 200, 300, 400, 520) comprenant :
une première composition (116, 216, 280), qui se transforme d'un premier état en un deuxième état, chacun ayant une couleur différente ; et
une deuxième composition (108, 208, 260), qui se dissout lorsqu'elle entre en contact avec l'exsudat de plaie et qui force la transformation de la première composition du premier état en le deuxième état lorsqu'elle entre en contact avec celle-ci,
où la deuxième composition (108, 208, 260) libère des ions d'hydrogène ou d'hydroxyde dans l'exsudat de plaie afin de modifier des niveaux d'analyte à l'intérieur d'une portion de l'exsudat de plaie, et de faire que la couleur du premier état change pour celle du deuxième état.

2. Le pansement pour plaie selon la revendication 1, où les première et deuxième compositions sont imprégnées dans des matières porteuses différentes à l'intérieur du dispositif.

3. Le pansement pour plaie selon la revendication 2, où les première et deuxième matières porteuses sont physiquement séparées par une matière d'espacement.

4. Le pansement pour plaie selon la revendication 1, où les première et deuxième compositions sont imprégnées dans une matière porteuse unique à l'intérieur du dispositif.

5. Le pansement pour plaie selon la revendication 4, où au moins une des première et deuxième compositions est encapsulée dans une barrière soluble qui se dissout lorsqu'elle entre en contact avec de l'exsudat de plaie afin de permettre une interaction entre les compositions.

6. Un pansement pour plaie selon la revendication 1, où le pansement pour plaie comprend une couche absorbante ayant une surface tournée vers la plaie et une surface opposée qui n'est pas tournée vers la plaie et où l'indicateur est positionné à l'intérieur du pansement de telle sorte que l'indicateur est en contact avec la surface qui n'est pas tournée vers la plaie de la couche absorbante.

7. Le pansement pour plaie selon n'importe laquelle des revendications 1 ou 6, où le pansement pour plaie a un bord périphérique et où l'indicateur s'étend vers l'extérieur depuis le bord périphérique.

8. Un pansement pour plaie selon la revendication 7, où l'indicateur s'étend depuis le bord périphérique du pansement afin de former une bague annulaire.
